(19) Europäisches Patentamt
European Patent Office
Office européen des brevets

(11) EP 3 398 639 A1

(12) EUROPEAN PATENT APPLICATION
published in accordance with Art. 153(4) EPC

(43) Date of publication:
07.11.2018 Bulletin 2018/45

(51) Int Cl.:
A61M 16/01 (2006.01)     A61M 16/00 (2006.01)
G05D 7/06 (2006.01)     G05B 11/42 (2006.01)

(21) Application number: 16880354.2

(22) Date of filing: 15.04.2016

(86) International application number:
PCT/CN2016/079347

(87) International publication number:
WO 2017/113546 (06.07.2017 Gazette 2017/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 29.12.2015 CN 201511017387

(71) Applicant: Beijing Aeonmed Co., Ltd.
Beijing 100070 (CN)

(72) Inventors:
• TIAN, Yongfeng
Beijing 100070 (CN)
• HAN, Wenlan
Beijing 100070 (CN)

(74) Representative: Hanna Moore + Curley
Garryard House
25/26 Earlsfort Terrace
Dublin 2, D02 PX51 (IE)

(54) FUZZY ADAPTIVE PID CONTROL-BASED CAPACITY CONTROL METHOD OF ANESTHESIA MACHINE

(57) A fuzzy adaptive PID control-based capacity control method of an anesthesia machine. The method comprises: obtaining a corresponding initial voltage value according to a predetermined flow value, and inputting the voltage value as a current voltage value into a flow sensor; obtaining a real-time flow value, and a difference value between the real-time flow value and the predetermined flow value, and inputting the two values into a fuzzy adaptive PID controller; calculating and outputting a voltage value by regulating the proportional-control coefficient, integral-control coefficient, and derivative-control coefficient of the PID controller; updating, by using the voltage value, the current voltage value and inputting the same into the flow sensor; and repeating the process until the flow value outputted by the flow sensor satisfies requirements. By means of the method, an anesthesia machine can output a stable and accurate tidal volume in the situations of different predetermined values and different patients.

Figure 1

## Description

## TECHNICAL FIELD

The present invention relates to the field of anaesthesia machines, and more specifically, to a fuzzy adaptive PID control-based capacity control method of an anesthesia machine.

## BACKGROUND OF THE INVENTION

The theoretical basis for a traditional PID control method of an anesthesia machine is a classical control theory based on a fixed mathematical model. However, since many factors such as human body physiological changes are time-varying nonlinear, the mathematical model of the anesthesia machine is actually a time-varying nonlinear model, and the traditional PID control method will lead to invalid control in the practical application. Therefore, in the practical application, a PID controller can be simplified as a system whose basic linearity and dynamic properties do not vary with time. In this way, the drawback of invalid control in the PID control method can be overcome, but the control effect is far from satisfactory.

## SUMMARY OF THE INVENTION

An objective of the present invention is to overcome the foregoing drawbacks of the PID controller in the existing anesthesia machine by proposing a fuzzy adaptive PID control-based capacity control method of an anesthesia machine. Considering that the traditional PID control can eliminate steady-state errors and accelerate dynamic response, the present method combines fuzzy control with the traditional PID controller, making best use of advantages and avoiding disadvantages. Thereby, the method not only has the advantages of flexible fuzzy control and good adaptability, but also has the characteristic of high precision of PID control.

To achieve the foregoing objective, the present invention provides a fuzzy adaptive PID control-based capacity control method of an anesthesia machine, the method comprises:

obtaining a corresponding initial voltage value according to a predetermined flow value, and inputting the voltage value as a current voltage value into a flow sensor; obtaining a real-time flow value, and a difference value between the real-time flow value and the predetermined flow value, and inputting the two values into a fuzzy adaptive PID controller; calculating and outputting a voltage value by regulating the proportional-control coefficient, integral-control coefficient, and derivative-control coefficient of the PID controller; updating, by using the voltage value, the current voltage value and inputting the same into the flow sensor; and repeating the process until the flow value outputted by the flow sensor satisfies requirements.

In the foregoing technical solution, the method specifically comprises:

step 1) obtaining a corresponding voltage value Eo by looking up a table according to a predetermined flow value Lo; and setting a current voltage value to be Eo;

step 2): inputting the current voltage value into an air suction valve, and obtaining a real-time flow value L by means of flow signal detection;

step 3): calculating a difference value $\Delta L$ between the real-time flow value L and the predetermined flow value $L_0$;

step 4): determining whether the difference value $\Delta L$ is less than a first threshold, if the result of the determination is positive, then proceeding to step 6); otherwise, proceeding to step 5);

step 5): inputting the real-time flow value L and the difference value $\Delta L$ obtained in step 3) into a fuzzy adaptive PID controller, calculating and outputting a new voltage value; and updating, by using the voltage value, the current voltage value, and returning to step 2); and

step 6): recording the current voltage value.

In the foregoing technical solution, step 5) specifically comprises:

step 5-1): determining whether $\Delta L$ is larger than a second threshold, if the result of the determination is positive, then regulating the proportional-control coefficient and the integral-control coefficient of the PID controller; and proceeding to step 5-2);

step 5-2): calculating a change rate $\dfrac{\Delta L}{L}$ of $\Delta L$;

step 5-3): determining whether $\dfrac{\Delta L}{L}$ is larger than a third threshold, if the result of the determination is positive, then regulating the derivative-control coefficient of the PID controller; and proceeding to step 5-4); and

step 5-4): calculating and outputting a voltage value by using the PID controller according to the real-time flow value L and the difference value $\Delta L$, updating the current voltage value, and returning to step 2).

The advantage of the present invention is that by means of the method of the present invention, an anesthesia machine can output a stable and accurate tidal volume in the situations of different predetermined values and different patients.

## BRIEF DESCRIPTION OF THE DRAWINGS

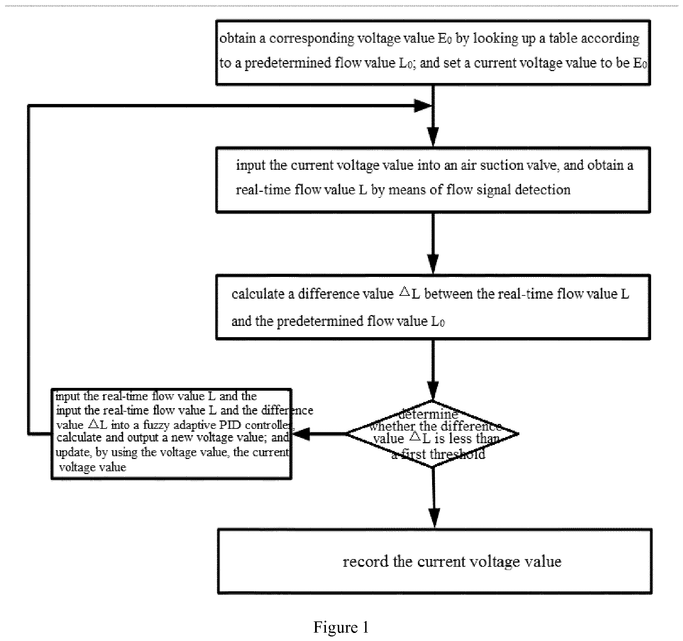

Fig. 1 is a flowchart of a fuzzy adaptive PID control-based capacity control method of an anesthesia machine according to the present invention.

## DETAILED DESCRIPTION OF THE INVENTION

The present invention is further described in detail with reference to the accompanying drawing and the specific example.

As shown in Fig. 1, a fuzzy adaptive PID control-based capacity control method of an anesthesia machine comprises:

step 1) obtaining a corresponding voltage value $E_0$ by looking up a table according to a predetermined flow value $L_0$; and setting a current voltage value to be $E_0$;

step 2): inputting the current voltage value into an air suction valve, and obtaining a real-time flow value L by means of flow signal detection;

step 3): calculating a difference value $\Delta L$ between the real-time flow value L and the predetermined flow value $L_0$;

step 4): determining whether the difference value $\Delta L$ is less than a first threshold, if the result of the determination is positive, then proceeding to step 6); otherwise, proceeding to step 5);

step 5): inputting the real-time flow value L and the difference value $\Delta L$ obtained in step 3) into a fuzzy adaptive PID controller, calculating and outputting a new voltage value; and updating, by using the voltage value, the current voltage value, and returning to step 2); specifically, the step comprising:

step 5-1): determining whether $\Delta L$ is larger than a second threshold, if the result of the determination is positive, then regulating a proportional-control coefficient and an integral-control coefficient of the PID controller; and proceeding to step 5-2);

step 5-2): calculating a change rate $\dfrac{\Delta L}{L}$ of $\Delta L$;

step 5-3): determining whether $\dfrac{\Delta L}{L}$ is larger than a third threshold, if the result of the determination is positive, then regulating a derivative-control coefficient of the PID controller; and proceeding to step 5-4); and

step 5-4): calculating and outputting a voltage value by using the PID controller according to the real-time flow value L and the difference value $\Delta L$, updating the current voltage value, and returning to step 2);

wherein through automatic adjustments of the various parameters, the stability of the PID controller can be improved, and a good dynamic response performance can be obtained;

step 6): recording the current voltage value.

## Claims

1. A fuzzy adaptive PID control-based capacity control method of an anesthesia machine, the method comprising: obtaining a corresponding initial voltage value according to a predetermined flow value, and inputting the voltage value as a current voltage value into a flow sensor; obtaining a real-time flow value, and a difference value between the real-time flow value and the predetermined flow value, and inputting the two values into a fuzzy adaptive PID controller; calculating and outputting a voltage value by regulating the proportional-control coefficient, integral-control coefficient, and derivative-control coefficient of the PID controller; updating, by using the voltage value, the current voltage value and inputting the same into the flow sensor; and repeating the process until the flow value outputted by the flow sensor satisfies requirements.

2. The fuzzy adaptive PID control-based capacity control method of an anesthesia machine according to claim 1, wherein the method specifically comprises:

step 1) obtaining a corresponding voltage value Eo by looking up a table according to a predetermined flow value Lo; and setting a current voltage value to be Eo;

step 2): inputting the current voltage value into an air suction valve, and obtaining a real-time flow value L by means of flow signal detection;

step 3): calculating a difference value $\Delta L$ between the real-time flow value L and the predetermined flow value Lo;

step 4): determining whether the difference value $\Delta L$ is less than a first threshold, if the result of the determination is positive, then proceeding to step 6); otherwise, proceeding to step 5);

step 5): inputting the real-time flow value L and the difference value $\Delta L$ obtained in step 3) into a fuzzy adaptive PID controller, calculating and outputting a new voltage value; and updating, by using the voltage value, the current voltage value, and returning to step 2); and

step 6): recording the current voltage value.

3. The fuzzy adaptive PID control-based capacity control method of an anesthesia machine according to claim 2, wherein step 5) specifically comprises:

step 5-1): determining whether $\Delta L$ is larger than a second threshold, if the result of the determination is positive, then regulating the proportional-control coefficient and the integral-control coefficient of the PID controller; and proceeding to step 5-2);

step 5-2): calculating a change rate $\dfrac{\Delta L}{L}$ of $\Delta L$;

step 5-3): determining whether $\dfrac{\Delta L}{L}$ is larger than a third threshold, if the result of the determination is positive, then regulating the derivative-control coefficient of the PID controller; and proceeding to step 5-4); and

step 5-4): calculating and outputting a voltage value by using the PID controller according to the real-time flow value L and the difference value $\Delta L$, updating the current voltage value, and returning to step 2).

obtain a corresponding voltage value $E_0$ by looking up a table according to a predetermined flow value $L_0$; and set a current voltage value to be $E_0$

input the current voltage value into an air suction valve, and obtain a real-time flow value L by means of flow signal detection

calculate a difference value $\triangle L$ between the real-time flow value L and the predetermined flow value $L_0$

input the real-time flow value L and the input the real-time flow value L and the difference value $\triangle L$ into a fuzzy adaptive PID controller; calculate and output a new voltage value; and update, by using the voltage value, the current voltage value

determine whether the difference value $\triangle L$ is less than a first threshold

record the current voltage value

Figure 1

5

# INTERNATIONAL SEARCH REPORT

| | International application No. |
|---|---|
| | PCT/CN2016/079347 |

**A. CLASSIFICATION OF SUBJECT MATTER**

A61M 16/01 (2006.01) i; A61M 16/00 (2006.01) i; G05D 7/06 (2006.01) i; G05B 11/42 (2006.01) i

According to International Patent Classification (IPC) or to both national classification and IPC

**B. FIELDS SEARCHED**

Minimum documentation searched (classification system followed by classification symbols)

A61M; G05D; G05B

Documentation searched other than minimum documentation to the extent that such documents are included in the fields searched

Electronic data base consulted during the international search (name of data base and, where practicable, search terms used)

CNABS; CNTXT; CNKI; VEN: proportion, integration, differentiation, anaesthe+, anesthe+, threshold, adaptive, deviation, fuzzy, rate, change, voltage, anesthesia, flow, gradient, self-adaption

**C. DOCUMENTS CONSIDERED TO BE RELEVANT**

| Category* | Citation of document, with indication, where appropriate, of the relevant passages | Relevant to claim No. |
|---|---|---|
| X | CN 102346493 A (BEIJING AEROSPACE CHANGFENG CO., LTD.) 08 February 2012 (08.02.2012) description, paragraphs [0027]-[0060], and figure 1 | 1-3 |
| A | CN 103071221 A (BEIJING AEROSPACE CHANGFENG CO., LTD.) 01 May 2013 (01.05.2013) the whole document | 1-3 |
| A | CN 102397607 A (BEIJING AEROSPACE CHANGFENG CO., LTD.) 04 April 2012 (04.04.2012) the whole document | 1-3 |
| A | CN 102397608 A (BEIJING AEROSPACE CHANGFENG CO., LTD.) 04 April 2012 (04.04.2012) the whole document | 1-3 |
| A | US 2012090611 A1 (GRABOI DAN et al.) 19 April 2012 (19.04.2012) the whole document | 1-3 |

☐ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 18 September 2016 | 10 October 2016 |

| Name and mailing address of the ISA | Authorized officer |
|---|---|
| State Intellectual Property Office of the P. R. China No. 6, Xitucheng Road, Jimenqiao Haidian District, Beijing 100088, China Facsimile No. (86-10) 62019451 | CHEN, Yang Telephone No. (86-10) 62085809 |

Form PCT/ISA/210 (second sheet) (July 2009)

**INTERNATIONAL SEARCH REPORT**
Information on patent family members

International application No.

PCT/CN2016/079347

| Patent Documents referred in the Report | Publication Date | Patent Family | Publication Date |
|---|---|---|---|
| CN 102346493 A | 08 February 2012 | None | |
| CN 103071221 A | 01 May 2013 | CN 103071221 B | 22 July 2015 |
| CN 102397607 A | 04 April 2012 | CN 102397607 B | 13 August 2014 |
| CN 102397608 A | 04 April 2012 | CN 102397608 B | 11 June 2014 |
| US 2012090611 A1 | 19 April 2012 | WO 2012051439 A1 | 19 April 2012 |

Form PCT/ISA /210 (patent family annex) (July 2009)